# EUROPEAN PATENT APPLICATION

(11) **EP 3 885 361 A1**
(43) Date of publication of application: **29.09.2021**
(21) Application number: 21159700.0
(22) Date of filing: 26.02.2021
(51) Int. Cl.: C07K 16/10, A61P 31/14, B01J 20/28, C07K 16/00, B01J 20/26

(54) **MONOCLONAL-TYPE SYNTHETIC ANTIBODIES OBTAINED BY MOLECULAR IMPRINTING AND THEIR USE FOR THE TREATMENT AND PREVENTIVE MEDICINE OF COVID-19**

(30) Priority: 27.02.2020 IT 202000004135; 09.06.2020 IT 202000013762
(71) Applicant: Macrofarm Srl, 87036 Rende (CS) (IT)
(72) Inventor: PUOCI, Francesco, 87100 Cosenza (CS) (IT); PARISI, Ortensia Ilaria, 87040 Castrolibero (CS) (IT)
(74) Representative: Giuliano, Natalia

(57) **Abstract**

Monoclonal-type synthetic antibodies, produced by Molecular Imprinting, capable of specifically recognizing and binding the receptor-binding domain (RBD) of Subunit S1 and/or Subunit S2 of SARS-CoV-2 Spike Protein, in which the receptor-binding domain RBD is responsible for host cell receptor recognition and binding, while Subunit S2 is involved in the viral and host membranes fusion.

## Description

The present invention relates to the technical field of antibodies and, more particularly, to the preparation of a monoclonal-type synthetic antibody, by Molecular Imprinting, capable of specifically recognizing and binding the receptor-binding domain (RBD) of the Subunit S1 and / or Subunit S2 of the Spike Protein of SARS-CoV-2 and its use for the prevention and treatment of infections due to the novel coronavirus SARS-CoV-2, initially called 2019-nCoV.

As it is known, a pandemic is currently underway caused by SARS-CoV-2 (severe acute respiratory syndrome coronavirus 2). COVID-19 is the name given to the disease associated with this virus. SARS-CoV-2 belongs to the coronavirus family which includes strains belonging to the common cold and SARS (Severe Acute Respiratory Syndrome). The mechanisms by which these pathogens evolve and what aggravates the diseases they cause are currently being studied. Many of the symptoms caused by SARS-CoV-2, such as acute respiratory syndrome, are similar to the symptoms caused by SARS-CoV. SARS-CoV emerged in 2002-2003 causing over 8000 confirmed cases of infection and about 800 deaths. SARS-CoV originated in bats and was transmitted to humans through an intermediate host, in particular palm civets. The source of the SARS-CoV-2 pandemic has been linked to a wholesale market in Wuhan, where it was initially confirmed that the majority of cases with onset prior to January 1st, 2020, were linked to the market. The market sells many species including snakes, seafood, marmots, birds and bats. Origination of SARS-CoV-2 from bats has been strongly supported, but the presumed intermediate host remains to be identified.
Coronaviruses are a large family of single-stranded enveloped RNA viruses and can be divided into four major genera. Both SARS-CoV and SARS-CoV-2 belong to the β-genus and recognize the same receptor, angiotensin-converting enzyme 2 (ACE2), in humans. The viral genome is about 27-32 kb, which encodes for both structural and non-structural proteins. Structural proteins are: membrane protein (M), envelope protein (E), nucleocapsid protein (N) and spike protein (S).
The coronavirus spike protein (S) is the primary determinant of viral tropism and is responsible for receptor binding and membrane fusion. It is a large glycoprotein that is present on the viral surface as a prominent trimer, and it is composed of two functional subunits: the S1 subunit, which contains a receptor-binding domain (RBD) responsible for ACE2 host cell receptor recognition and binding, and the S2 subunit, which is involved in the viral and host membranes fusion. These two processes, which represent the initial steps in the coronavirus infection cycle, are crucial in determining host specificity, tissue tropism and transmission capacity. SARS-CoV-2 RBD contains a core and a receptor-binding motif (RBM). RBM mediates contact with ACE2. The surface of ACE2 contains two virus-binding hotspots that are essential for SARS-CoV-2 binding. Various studies report that the key amino acids of RBM that interact with the ACE2 receptor are L455, F486, Q493, S494, N501 and Y505 (J. Luan et al. Spike protein recognition of mammalian ACE2 predicts the host range and an optimized ACE2 for SARS-CoV-2 infection. Biochemical and Biophysical Research Communications 526 (2020) 165e169). The interaction of RBM amino acids with ACE2 hot spots allows the virus to infect cell.
Therefore, the spike protein, which is involved in viral recognition and binding to human ACE2 playing a key role also in human-to-human transmission of this novel coronavirus, represents the common and primary target for the development of antibodies, vaccines and therapeutic agents.

At present, there are no specific treatments for this novel coronavirus, therefore different strategies are being explored.

Passive immunization with antibodies capable of recognizing specific regions of the viral particle could avoid the replication of the virus in the body. In fact, based on previous treatments used for SARS, the administration of plasma or hyperimmune immunoglobulins derived from patients that contain a significant amount of antibodies can reduce the viral load and mortality associated with the disease.

A solution is then described in the paper Xialong Tian et al.: "Potential binding of 2019 novel coronavirus spike protein by a SARS coronavirus-specific human monoclonal antibody", Emerging Microbes & Infections, XP055736759, reporting a human monoclonal antibody (CR3022) specific for SARS-CoV, isolated from the blood of a convalescent SARS patient and capable of binding also the RBD domain of SARS-CoV-2.

Another solution is described in the paper Damian Garde: "In race to develop coronavirus treatment, Regeneron sees an inside track", 5 February 2020, XP055740222, which reports human antibodies, produced by genetically engineered mice, directed against the SARS-CoV-2 spike protein. The research described is still at an early stage in which mice are immunized to obtain human antibodies capable of fighting SARS-CoV-2 infection.

Biological antibodies, however, are characterized by several disadvantages such as limited stability, unsuitable pharmacokinetics, inefficient tissue penetration, high production costs.

An interesting alternative to conventional biological antibodies is represented by synthetic antibodies with a polymeric nature.

In this context, Molecular Imprinting is a promising technology for the preparation of synthetic antibodies based on Molecularly Imprinted Polymers (MIPs) capable of selectively recognizing and binding a target molecule called "template". Being synthetic materials, MIPs are resistant, stable in different conditions and easily available thanks to an economical, reproducible and fast production process compared to biological antibodies.

The imprinting procedure involves the polymerization of functional monomers and cross-linking agents around the target molecule (template).

The selective recognition abilities of the imprinted polymers are due to the formation of a complex between the target molecule and the selected functional monomers during the pre-polymerization step. Therefore, the choice of the monomers represents a crucial point in the preparation of MIPs and it is based on their ability to establish interactions with the functional groups of the template molecule in a covalent or non-covalent way. Once the polymerization reaction has taken place, the template is extracted leading to a porous crosslinked polymeric matrix containing binding holes fitting size, shape and functionalities of the target compound.

Monoclonal-type synthetic antibodies, made from polymeric imprinted nanoparticles, represent an alternative to the traditional antibodies, which require an expensive production procedure and are often unreliable due to their restricted stability. Being synthetic materials, MIPs are robust, physically and chemically stable in a wide range of conditions, including temperature and pH, and more easily available due to a low-cost, reproducible and relatively fast and easy preparation compared to the biological counterpart. Therefore, this kind of materials combines the robustness of polymers with the selectivity of natural receptors and could find applications in several fields, including separation, catalysis and drug delivery, as sensors, synthetic biomimetic receptors and recognition elements in bioanalytical assays.

An example of synthetic antibodies obtained by Molecular Imprinting is reported in the publication "Molecurarly imprinted polymer nanoparticles as pontential synthetic antibodies for immunoprotection against HIV" [ACS Appl. Mater. Interfaces 2019, 11, 9824-9831] in which is described the preparation and characterization of synthetic antibodies based on Molecularly Imprinted Polymer Nanoparticles (MIP-NPs which allow the recognition and binding of the highly conserved peptide motif SWSNKS (3S), an epitope of the glycoprotein 41 (gp41) of HIV type 1 virus. Specifically, the 3S motif is implicated in the depletion of CD4 + T lymphocytes with consequent inhibition of the cell-mediated immune response and increased risk of infections and tumors. However, the antibodies described in this paper are not directed against the spike protein of a coronavirus, but are specific for the 3S peptide of HIV.

Then, the purpose of the present invention is to provide synthetic antibodies able to specifically recognize and bind the receptor-binding domain (RBD) of Subunit S1 and / or Subunit S2 of the Spike Protein of the novel coronavirus SARS-CoV-2, in the aim to block the function of spike protein, having a greater stability and scalable and low-cost synthetic strategy compared to biological antibodies.

According to the present invention, a synthetic monoclonal-type antibody is realized, as defined in claim 1.

To better understand the present invention, a preferred embodiment is now described, as a non-limiting example, in the attached drawing, in which:

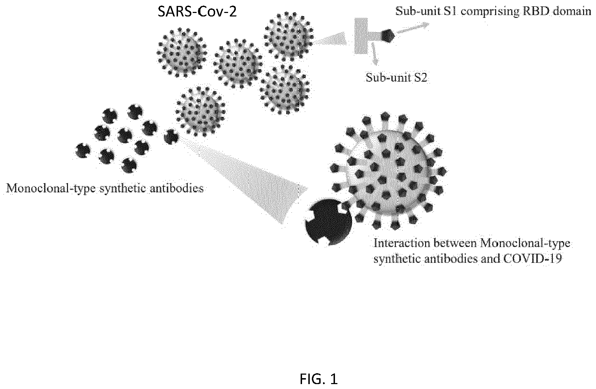

- Figure 1 shows the interaction between monoclonal-type synthetic antibodies and SARS-CoV-2, according to the invention. In particular, Figure 1 shows the interaction between monoclonal-type synthetic antibodies and the receptor-binding domain (RBD) responsible for the recognition and binding of the coronavirus with the host cell. According to an aspect of the invention, the monoclonal-type synthetic antibody is developed by Molecular Imprinting and is therefore able to specifically recognize and bind the receptor-binding domain (RBD) of the S1 subunit and / or S2 Subunit of the Spike Protein of the novel coronavirus SARS-CoV-2.

The RBD domain is, in fact, responsible for the recognition and binding of SARS-CoV-2 with the receptor present on the surface of the host cell, while the S2 subunit is responsible for the fusion of the membranes.

To explain better, the synthetic antibodies object of the present invention consists of molecularly imprinted polymer nanoparticles prepared by Molecular Imprinting technology. The imprinting procedure involves the polymerization of functional monomers and cross-linking agents around the target molecule (template), in this case represented by specific portions of SARS-CoV-2 spike protein, in order to obtain a highly cross-linked polymeric matrix. The choice of the monomers to be used depends on their ability to establish interactions with the functional groups of the template. Once the polymerization has taken place, the extraction of the template allows to obtain a macroporous polymeric matrix that has highly specific and selective cavities that are complementary to the target template in terms of size, shape and functional groups. The selective recognition properties of MIPs are due to the formation of a complex between template and functional monomers during the pre-polymerization step. The monomers chosen must, therefore, have functional groups capable of interacting with the template molecule through covalent or non-covalent interactions.

Then, synthetic antibodies based on MIPs are, therefore, not biomacromolecules, but are polymeric nanoparticles capable of recognizing the receptor-binding domain (RBD) of the S1 subunit and / or S2 Subunit of the Spike Protein of the novel coronavirus SARS-CoV-2.

According to an aspect of the invention, the monoclonal-type synthetic antibodies are prepared using as template the receptor-binding domain (RBD) of the S1 subunit and / or S2 Subunit of the Spike Protein of the novel coronavirus SARS-CoV-2.

According to an aspect of the invention, the monoclonal-type synthetic antibodies are prepared using as template an amino acid sequence present in the receptor-binding domain (RBD) of the S1 subunit of SARS-CoV-2, preferably the receptor-binding motif (RBM) or its amino acid sequences which contain the amino acids that interact with ACE2 hot spots. The amino acid sequences are selected from the group consisting of:
RVQPTESIVRFPNITNLCPFGEVFNATRFASVYAWNRKRISNCVADYSVLYNSA SFSTFKCYGVSPTKLNDLCF,
TNVYADSFVIRGDEVRQIAPGQTGKIADYNYKLPDDFTGCVIAWNSNNLDSKVGGNYNY LYRLFRKSNLKPFER,
DISTEIYQAGSTPCNGVEGFNCYFPLQSYGFQPTNGVGYQPYRVVVLSFELLHAPATVC GPKKSTNLVKNKCVNF, NSNNLDSKVGGNYNYLYRLFRKSNLKPFERDISTEIYQAGSTPCNGVEGFNCYFPLQSY GFQPTNGVGYQPY, NSNNLDSKVGGNYNYLYRLFRKSN, LKPFERDISTEIYQAGSTPCNGVE, GFNCYFPLQSYGFQPTNGVGYQPY, and their fragments.

According to one aspect of the invention, the template will preferably be the receptor-binding motif (RBM) or its amino acid sequences that contain the amino acids that interact with the ACE2 hot spots:
NSNNLDSKVGGNYNYLYRLFRKSNLKPFERDISTEIYQAGSTPCNGVEGFNCYF PLQSYGFQPTNGVGYQPY, NSNNLDSKVGGNYNYLYRLFRKSN, LKPFERDISTEIYQAGSTPCNGVE, GFNCYFPLQSYGFQPTNGVGYQPY, and their fragments.

According to one aspect of the invention, monoclonal-type synthetic antibodies are developed using different synthetic strategies including solid phase synthesis, precipitation polymerization, emulsion polymerization, reverse microemulsion polymerization, suspension polymerization, solution polymerization, RAFT polymerization

According to one aspect of the invention, the monoclonal-type synthetic antibodies are developed using different functional monomers, cross-linking agents, initiators and surfactants including acrylamide, methacrylamide, N-isopropylacrylamide, acrylic acid, methacrylic acid, methyl methacrylate, 2-hydroxyethyl methacrylate, N, N'-methylene-bis-acrylamide, ethylene glycol dimethacrylate, trimethylolpropane trimethacrylate, ammonium persulfate, potassium persulfate, sodium persulfate, 2-2'-azobisisobutyronitrile, benzoyl peroxide, N, N, N, N-tetramethylethylenediamine salt, dioctyl sulfosuccinate sodium salt, tween20, tween40, tween60, tween80, span80, laureth-4, and derivatives.

According to one aspect of the invention, the monoclonal-type synthetic antibodies are modified with lipopolysaccharides (LPS) in order to stimulate the response by the immune system.

According to one aspect of the invention, the monoclonal-type synthetic antibodies have size between 10 and 60 nm.

According to one aspect of the invention, the monoclonal-type synthetic antibodies can be administered by any suitable method of administration, preferably parenteral and inhalation administration.

According to one aspect of the invention, the monoclonal-type synthetic antibodies are loaded with drugs able to stimulate the immune response against viral infections by acting as ligands for the Toll-Like Receptors TLR7 and TLR8, including gardiquimod, imiquimod and R848 (Resiquimod) .

According to one aspect of the invention, the synthetic mnoclonal-type antibodies are loaded with antiretroviral drugs already on the market and potentially useful in the treatment of SARS-CoV-2 infections, including indinavir, saquinavir, lopinavir, ritonavir, umifenovir, darunavir, remdesivir, and new antiviral drugs specific for the novel coronavirus.

According to one aspect of the invention, the monoclonal-type synthetic antibodies are capable of acting as drug-free therapeutics able to counteract SARS-CoV-2 infection by antagonizing the interaction between the RBD domain of the coronavirus and the receptor present on the surface of the host cell and / or preventing the membrane fusion process.

According to one aspect of the invention, monoclonal-type synthetic antibodies are the basis for the development of systems for the preventive medicine of SARS-CoV-2 infections.

An embodiment of the present invention has been described below.

### Example 1.

The monoclonal-type synthetic antibodies were prepared by Molecular Imprinting, a very promising technology for the synthesis of Molecularly imprinted polymers (MIPs) characterized by high selective recognition capabilities for a target molecule called template.

The imprinting procedure involves the polymerization of suitable functional monomers and cross-linking agents around the template (receptor-binding domain (RBD) of Subunit S1 of the novel coronavirus SARS-CoV-2) in order to obtain a highly cross-linked polymeric matrix. The choice of monomers depends, in fact, on their ability to establish interactions with the functional groups of the template. Once the polymerization has taken place, the extraction of the template allows to obtain a macroporous polymeric matrix that has highly specific and selective cavities that are complementary to the target template in terms of size, shape and functional groups.

The selective recognition properties of MIPs are due to the formation of a complex between template and functional monomer during the pre-polymerization step. The monomers chosen must, therefore, have functional groups capable of interacting with the template molecule through covalent or non-covalent interactions.

In order to study the recognition properties and selectivity of the monoclonal-type synthetic antibodies prepared, binding studies were conducted in which known quantities of the imprinted polymeric nanoparticles, and of the corresponding non-imprinted control, were incubated with a solution of template with known concentration.

The selectivity tests were performed under the same conditions, but using a solution with a known concentration of receptor-binding domain (RBD) of Subunit S1 of SARS CoV, that is a molecule structurally similar to the template used during polymerization. The results obtained from the binding studies showed the selective recognition properties of monoclonal-type synthetic antibodies with binding percentages of the template between 20% and 100%.

Advantageously, according to the invention, the monoclonal-type synthetic antibodies allow the development of systems for the treatment of SARS-CoV-2 infections.

Advantageously, according to the invention, the monoclonal-type synthetic antibodies allow the development of systems capable of acting as drug-free therapeutics against SARS-CoV-2 infection by antagonizing the interaction between the RBD domain of the coronavirus and the receptor present on the surface of the host cell and / or preventing the membrane fusion process.

Advantageously, according to the invention, the monoclonal-type synthetic antibodies allow the development of systems for the preventive medicine of SARS-CoV-2 infections.

Finally, it is clear that the monoclonal-type synthetic antibody and its use here described and illustrated can be subject to modifications and variations without departing from the scope of the present invention, as defined in the claims.

## Claims

1. Monoclonal-type synthetic antibodies, having size between 10 and 60 nm, produced by Molecular Imprinting, capable of specifically recognizing and binding the receptor-binding domain (RBD) of Subunit S1 and/or Subunit S2 of SARS-CoV-2 Spike Protein, in which the receptor-binding domain (RBD) is responsible for host cell receptor recognition and binding, while Subunit S2 is involved in the viral and host membranes fusion.

2. Monoclonal-type synthetic antibodies, according to claim 1, capable of specifically recognizing and binding amino acid sequences present in the receptor-binding domain (RBD) of the S1 Subunit of SARS-CoV-2 Spike Protein, preferably the receptor-binding motif (RBM) or its amino acid sequences, responsible for host cell receptor recognition and binding.

3. Monoclonal-type synthetic antibodies, according to claim 1, in which the template is the receptor-binding domain (RBD) of Subunit S1 and/or Subunit S2 of SARS-CoV-2 Spike Protein, preferably an amino acid sequence present in the receptor-binding domain (RBD) of the S1 subunit of SARS-CoV-2, preferably the receptor-binding motif (RBM) or its amino acid sequences which contain the amino acids that interact with ACE2 hot spots. The amino acid sequences are selected from the group consisting of:
RVQPTESIVRFPNITNLCPFGEVFNATRFASVYAWNRKRISNCVADYSVLYNSA SFSTFKCYGVSPTKLNDLCF,
TNVYADSFVIRGDEVRQIAPGQTGKIADYNYKLPDDFTGCVIAWNSNNLDSKVG GNYNYLYRLFRKSNLKPFER,
DISTEIYQAGSTPCNGVEGFNCYFPLQSYGFQPTNGVGYQPYRVVVLSFELLHA PATVCGPKKSTNLVKNKCVNF,
NSNNLDSKVGGNYNYLYRLFRKSNLKPFERDISTEIYQAGSTPCNGVEGFNCYF PLQSYGFQPTNGVGYQPY, NSNNLDSKVGGNYNYLYRLFRKSN, LKPFERDISTEIYQAGSTPCNGVE, GFNCYFPLQSYGFQPTNGVGYQPY, and their fragments.

4. Monoclonal-type synthetic antibodies, according to claim 1, in which the polymerization takes place through synthetic strategies selected from the group consisting of: solid phase synthesis, precipitation polymerization, emulsion polymerization, reverse microemulsion polymerization, suspension polymerization, solution polymerization, RAFT polymerization.

5. Monoclonal-type synthetic antibodies according to claim 1, in which polymerization takes place through the use of functional monomers, cross-linking agents, initiators and surfactants selected from the group consisting of: acrylamide, methacrylamide, N-isopropylacrylamide, acrylic acid, methacrylic acid, methyl methacrylate, 2-hydroxyethyl methacrylate, N,N'-methylen-bis-acrylamide, ethylene glycol dimethacrylate, trimethylolpropane trimethacrylate, ammonium persulfate, potassium persulfate, 2-2'-azobis-isobutyrronitrile, benzoyl peroxide, N,N,N,N-tetramethylethylenediamine, dioctyl sulfosuccinate sodium salt, tween20, tween40, tween60, tween80, span80, laureth-4, and derivatives.

6. Monoclonal-type synthetic antibodies, according to one of previous claims, modified with lipopolysaccharides (LPS) in order to stimulate the response of the immune system.

7. Monoclonal-type synthetic antibodies, according to one of previous claims, loaded with drugs capable of stimulating the immune response against viral infections by acting as ligands for the Toll-Like Receptors TLR7 and TLR8, selected from the group consisting of: gardiquimod, imiquimod and R848 (Resiquimod) .

8. Monoclonal-type synthetic antibodies, according to one of previous claims, loaded with antiretroviral drugs potentially useful in the treatment of 2019-nCoV infections, selected from the group consisting of:indinavir, saquinavir, lopinavir, ritonavir, umifenovir, darunavir, remdesivir, and new antiviral drugs specific for novel coronavirus SARS-CoV-2.

9. Monoclonal-type synthetic antibodies, according to one of previous claims, and their use as drug-free therapeutics able to counteract SARS-CoV-2 infection by antagonizing the interaction between the RBD domain of the coronavirus and the receptor present on host cell surface and / or preventing the membrane fusion process.

10. Monoclonal-type synthetic, antibodies according to one of previous claims, and their use against different coronaviruses.

11. Monoclonal-type synthetic antibodies, according to claim 1, which can be administered by any suitable method of administration, preferably parenteral and inhalation administration.
